# EUROPEAN PATENT APPLICATION

(11) **EP 1 669 077 A1**
(43) Date of publication of application: **14.06.2006**
(21) Application number: 03750251.5
(22) Date of filing: 18.09.2003
(51) Int. Cl.: A61K 31/7008, A61K 31/43, A61K 31/4704, A61K 31/545, A61K 31/65, A61K 31/7036, A61K 31/7048, A61P 31/04

(54) **ANTIBACTERIAL COMPOSITIONS OF N-ACETYL-D-AMINOGLYCOSAMINE AND ANTIBIOTICS**

(71) Applicant: Third Military Medical University Chinese People's Liberation Army P.R. of China, Chongqing 400038 (CN); Bio-Wave Institute of Suzhou Hi-Tech New District Corporation, Ltd, Jiangsu 215011 (CN); Beijing Sino-Hongkong Dafu Science & Technology of Biowave Co., Ltd., Beijing 101200 (CN)
(72) Inventor: XU, Qiwang, Shapingba District, Chongqing 400038 (CN); LIU, Junkang, Shapingba District, Chongqing 400038 (CN); YUAN, Zetao, Shapingba District, Chongqing 400038 (CN)
(74) Representative: Adorno, Silvano
(86) International application number: PCT/CN2003/000793
(87) International publication number: WO 2005/025582

(57) **Abstract**

The use of the combination of N-acetyl-D-glucosamine and antibiotics for the preparation of antibacterial drugs is disclosed. In the therapies of infections with antibiotics, the pathogens may be changed into slender shaped forms called cryptic growth cells (CGCs), CGCs can colonize and thereby physiological drug resistance arises. In the meantime, normal bacteria colonies in the body may be also changed into CGCs upon administration of antibiotics. These changes result in complications after the therapies, such as disorder of bacteria colonies in the body, disorder of GI functions and other chronic diseases. The combination of antibiotics and N-acetyl-D-aminoglycosamine can prevent of CGC, and the complications after antibiotics therapy.

## Description

### TECHNICAL FIELD

The present invention relates to the use of N-acetyl-D-glucosamine and pharmaceutically acceptable salts, specifically relates to compound antibacterial drugs comprising N-acetyl-D-glucosamine and various antibiotics, and the use of N-acetyl-D-glucosamine in the manufacture of compound antibacterial drugs.

### BACKGROUND ART

Since the antibiotics were invented as a powerful weapon against the infections of pathogenic microorganisms, millions people are not threaten by such infections. However, with the lapse of time, the kind and dose of antibiotics increase gradually for combating widely occurred drug-resistant bacteria, which results in little effect on treating drug-resistant bacteria and hospital infections, while this causes various mutations of bacteria, including bacterial cryptic growth cells (CGC) as proposed in the present invention that is a new adequate form of bacteria in unadvantageous environments. Bacterial mutations result in changing normal bacterial flora into conditioned pathogenic bacteria, cause dysbacteriosis and hospital infections. The generation and colonization of bacterial CGC are closely related with irritable bowel syndrome and functional disorder of intestine, and these were confirmed in clinical investigations.

Even since a long time ago, bacteria are deemed as unicellular organism, and each bacterium independently performs life action, i.e., interaction usually does not occur between each other. However, researches in recent years indicate that bacterial single cell is different from bacterial population, in particular, as for interaction between bacteria and other organisms, the effects caused by bacteria actually are effects caused by bacterial colony as a whole, which acts like multicellular organism (Shapiro, J.A., "Bacteria as multicellular organism", *Scientific American,* 1988, 256: 82-89). According to the further study by the inventors of the present invention, bacteria may generate wave-like bacterial colonial pattern and exhibit organized vital activity phenomena under appropriate culturing conditions. This phenomena is interesting for discovering bacterial bionomy, and the inventors of the present invention name this phenomena as "bio-wave" (Xu Qiwang, et al., Population, cycle and wave of microorganism growth, *Ziran Zazhi (Chinese Journal of Nature),* 1992, 15(3):195-197), and studied it (Liu Junkang, et al., Study on bio-wave mechanism, *Zhongguo Weishengtaixue Zazhi (Chinese Journal of Microecology*), 1994, 6(6):40-46; Xu Qiwang, et al., Study on bio-wave non-equilibrium mechanism, *Xibei Daxue Xuebao (Journal of Northwest University)* (Natural Science Edition), 1997, 27 (Supplement): 320-325).

During the inventor's study on bio-wave theory, it was found that bacterial colony in waveform was in the alternative state of promotion and inhibition of bacterial growth. In the inhibition part of bacterial growth, bacteria did not divide and had a slender shape and a low metabolism level, but had strong action capability, which is called as "cryptic growth cell" (CGC) (Deng Guohong, et al., Observation of acyclic change of *Bacillus pyocyaneus* living in water, *Disan Junyidaxue Xuebao (ACTA Academiae Medicinae Militaris Tertiae),* 1997, 19(3): 197-201). In the further study on human pathology and physiology, it was found that bacteria abundantly changed into CGCs in the environment comprising antibacterial substances, and bacteria in the form of cryptic growth cells were very insensitive to antibiotics, and even a large amount of antibiotics did kill them (Deng Guohong, Effects of antibacterial drugs on bacterial CGCs, *Daziran Tansuo (Discovery of Nature),* 1999, 18(69): 67-68). Once antibiotics were eliminated or concentration thereof decreased to a level lower than MIC, bacteria adapted the environmental change and appeared corresponding changes, i.e., bacteria broke quickly to form individual cells that aggregated together to form bacterial colony. Experimental study indicated that antibiotics could induce the formation of CGCs of Gram-positive or Gram-negative bacteria, such as *E. coli,* proteus valgaris, *salmonella,* and Dysentery bacilli, and in vivo tests of both animal and human had proven that this effect was consistent with that of in vitro tests (Wang Zhenwei, et al., Observation on bio-spin of cryptic growth cells, *Zhongguo Gonggong Weisheng* (*China Public Health),* 2000, 16(5): 1-3; Liu Junkang et al., Turbulence under the microscope, *Journal of Biological Physics,* 2000, 00(1): 1-7).

The bacterial CGCs concerned in the present invention, whether they are formed by any bacterium, cause damage on human body. It had been disclosed that bacteria in slender shape occurred in different ecology environments had a low level of physiological metabolism and a changed toxicity, and caused damages on organism greatly different from the damages caused by bacteria in vegetative form (Guo Gang, Huang Chunji, et al., Comparative study on antibiotic Sub-MICs induced slender bacteria and cryptic growth cells, *Zhonghua Liuxingbingxue Zazhi (Chinese Journal of Epidemiology),* 1996, 17(3-C)). However, this difference did not draw any attention. The conversion of bacteria to cryptic growth cells leads a gradually increasing dose of antibiotics, while the effects thereof decreases gradually, so that the possibility of misdiagnosis and drug abuse increases, which may cause the aggravation of disease and even the delay of treatment and may lead to a large number of drug adverse reactions.

According to the inventors' long period study, it is proven that bacterial CGCs are caused by the contact with chemical substances, especially the wide use of antibiotics that result in the CGC change of bacteria in intestinal tract. It is disclosed that chronic diarrhea, chronic gastrointestinal dysfunction, especially irritable bowel syndrome (IBS) that greatly affect human, are caused by CGC directly or indirectly (Xu Qiwang, Cryptic growth cell pathogenesis of irritable bowel syndrome, *Kexue (Science Journal of China),* 1998, 10: 59-61).

During the inventors' study on bio-wave theory, it is found that this wave has inherent regulatory mechanism and the bio-wave can be regulated by chemical substances. By purification and identification, one of such substances is N-acetyl-D-glucosamine, and its promoting wave function has been confirmed (Huang Hui, et al., Experimental analysis of bacterial bio-wave regulating factor, *Disan Junyidaxue Xuebao (ACTA Academiae Medicinae Militaris Tertiae),* 1999, 21(3): 178-180).

N-acetyl-D-glucosamine (2-acetylamino-2-deoxy-D-glucose; N-acetyl-D-(+)- glucosamine; GlcNAc; CAS No. 7512-17-6) is a known compound of the following formula.

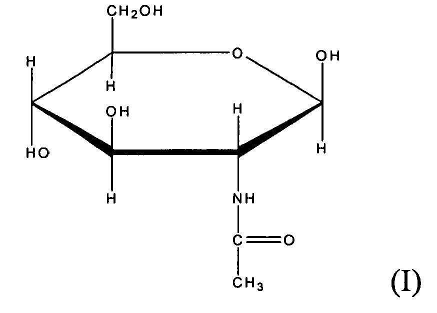

It is disclosed that N-acetyl-D-glucosamine is used in the treatment of diseases such as pericementitis (WO91/02530A1), intestinal inflammation (WO99/53929A1), cornea disease (JP10-287570A2), hypertrophy of the prostate (US5,116,615), etc., in the preparation of vaccines for preventing and treating microorganism infections (WO97/18790A3), and in cosmetology (JP59-013708A2), shampoo preparation (JP2-11505A2), etc. In recent years, the inventors deeply studied its wave-promoting function and filed the Chinese patent application (CN1156027A) that relates to its use as new drug for the treatment of IBS and the Chinese patent application (Chinese patent application number: 01104884.0) that relates to its use as therapeutic agent against bacterial colonization. However, it is not reported yet that N-acetyl-D-glucosamine can be used for the treatment of antibiotics induced bacterial CGC and diseases caused thereby, and it is not reported that N-acetyl-D-glucosamine is applied to bacteria in order to enhance antibiotic effects.

### DISCLOSURE OF THE INVENTION

When the inventors studied the promoting wave function of N-acetyl-D-glucosamine, we have surprisingly found that N-acetyl-D-glucosamine effectively prevented the conversion of bacteria into cryptic growth cells in the presence of antibiotics, so that the effects of various antibiotics were improved significantly. Thereby, the present invention is achieved.

In other words, the present invention relates to the antibacterial use of N-acetyl-D-glucosamine and pharmaceutically acceptable salts thereof. Concretely, the present invention relates to compound antibacterial drugs comprising N-acetyl-D-glucosamine and antibiotics and to the use of N-acetyl-D-glucosamine in the manufacture of compound antibacterial drugs. The present invention relates to the use of compositions comprising N-acetyl-D-glucosamine and antibiotics in the manufacture of a medicament for preventing or treating irritable bowel syndrome, in vivo dysbacteriosis, functional disorder of intestine, etc.

In addition, the present invention relates to a method for enhancing therapeutic effect of antibiotics comprising administering a therapeutically effective amount of N-acetyl-D-glucosamine and a therapeutically effective amount of antibiotics to a patient in need, and to a method for the treatment of diseases caused by bacterial infections or pathogenic proliferation that can be treated by antibiotics comprising administering a therapeutically effective amount of N-acetyl-D-glucosamine and a therapeutically effective amount of antibiotics to a patient in need.

The present invention further relates to a method for preventing or treating irritable bowel syndrome, in vivo dysbacteriosis, functional disorder of intestine, etc. comprising administering a therapeutically effective amount of N-acetyl-D-glucosamine and a therapeutically effective amount of antibiotics to a patient in need.

### DETAILED DESCRIPTION OF THE INVENTION

The present invention is illustrated as follows in detail.

In the drugs/compositions of the present invention that comprise N-acetyl-D-glucosamine and antibiotics, N-acetyl-D-glucosamine has the following formula.

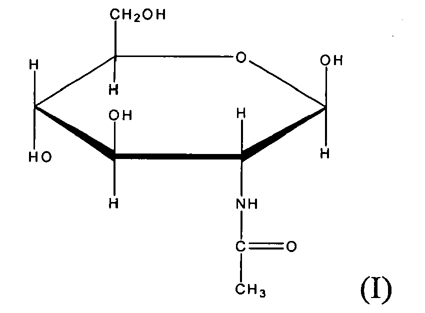

The physical/chemical parameters of N-acetyl-D-glucosamine are as follows:
Molecular formula: C₈H₁₅NO₆
Molecular weight: 221.21 (precise molecular weight: 221.2096)
Melting point: 201-204°C
N-acetyl-D-glucosamine can be from various sources. As for its preparation, it is usually prepared by chemical synthesis or semi-synthesis in China and foreign countries, and it is sometime prepared directly according known methods. For example, WO97/31121 discloses a method for preparing N-acetyl-D-glucosamine from chitin polysaccharide by enzyme method; JP6-3273493A has discloses a method comprising partially hydrolyzing chitin polysaccharide to obtain N-acetyl-chitose, and then treating it with an enzyme to obtain N-acetyl-D-glucosamine.

In the drugs/compositions of the present invention that comprise N-acetyl-D-glucosamine and antibiotics, N-acetyl-D-glucosamine can be in the form of freebase or pharmaceutically acceptable salts thereof. The pharmaceutically acceptable salts of N-acetyl-D-glucosamine are, for instance, the salts formed with inorganic acids, such as hydrochloride, hydrobromide, borate, phosphate, sulfate, hydrosulfate and hydrophosphate, and the salts formed with organic acids, such as citrate, benzoate, ascorbate, methyl sulfate, naphthalene-2-sulfonate, picrate, fumarate, maleate, malonate, oxalate, succinate, acetate, tartrate, mesylate, tosylate, isethionate, α-ketoglutarate, α-glyceryl phosphate and glucose-1-phosphate and so on. The said freebase or pharmaceutically acceptable salts are prepared according to known methods or commercially available from markets.

In the drugs and compositions of the present invention that comprise N-acetyl-D-glucosamine and antibiotics, antibiotics refer to substances that can be applied to human or other mammalians and exhibit chemically therapeutic effects against microorganisms such as bacteria, mycoplasma, chlamydia, etc. The sources of antibiotics of the present invention are not limited, and they can be from fermentation, semi-synthesis or full-synthesis. For example, they can be antibiotics obtained by fermentation of microorganisms (molds, antinomycetes, etc.) that produce antibiotics, or semi-synthetic antibiotics obtained by modifying structure of antibiotics from fermentation that have the same or similar parent cores, or full-synthetic antibiotics having structure similar to conventional antibiotics, or various total synthetic chemotherapeutic drugs such as quinolones that conventionally are deemed as antibiotics.

Once the antibiotics of the present invention induce bacteria to generate CGCs when they are applied to bacteria, they can be used in the compositions/drugs of the present invention.

In the present invention, the usable antibiotics include, but are not limited to: aminoglycoside antibiotics, penicillin group antibiotics, cephalosporin group antibiotics, β-lactam antibiotics other than penicillins and cephalosporins, chloramphenicol group antibiotics, lincomycin group antibiotics, macrolide group antibiotics, quinolones, tetracyclines, etc.

The aminoglycoside antibiotics of the present invention are antibiotics having aminoglycoside structure. For example, they are, but not limited to streptomycin (II), kanamycin, amikacin, etc., which are produced by streptomycetes or by semi-synthesis based thereon, or gentamycin, sagamicin, etc. which are produced by micromonosporaceae.

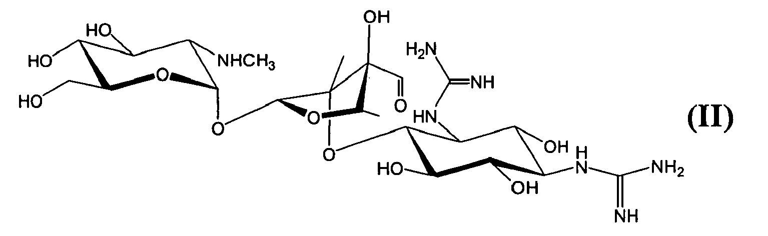

The penicillin group antibiotics of the present invention refer to natural or semi-synthetic antibiotic monomers having penicillanic acid parent cores structure (III) or various pharmaceutically acceptable salts thereof. For example, they are, but not limited to natural penicillins, such as penicillin G, penicillin V, etc.; and semi-synthetic penicillins, such as ampicillin, carbenicillin, amoxicillin, etc.

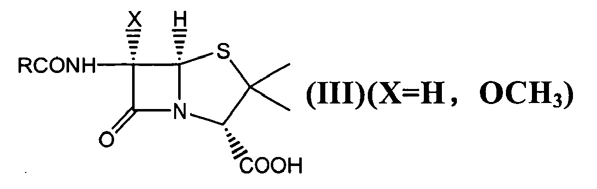

The cephalosporin group antibiotics of the present invention refer to synthetic, semi-synthetic or natural antibiotics having cephalosporanic acid (IV) or oxacephem (V) parent cores. For example, they are, but not limited to cefalexin, cefradine, cefaclor, cefuroxime, cefotaxime, latamoxef, etc.

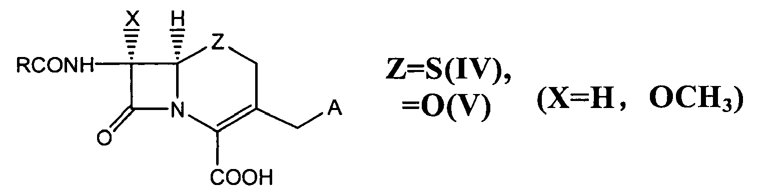

The β-lactam antibiotics other than penicillins and cephalosporins of the present invention refer to synthetic, semi-synthetic or natural antibiotics having β-lactam ring structure (VI) but not having penicillin or cephalosporin structure. For example, they are, but not limited to nocardicin, thiomycin and imipenem having carbapenem structure (VII); sulbactam, tazobactam, sultamicillin (ampicillin + sulbactam); clavulanic acid having oxapenam structure (IX), etc.

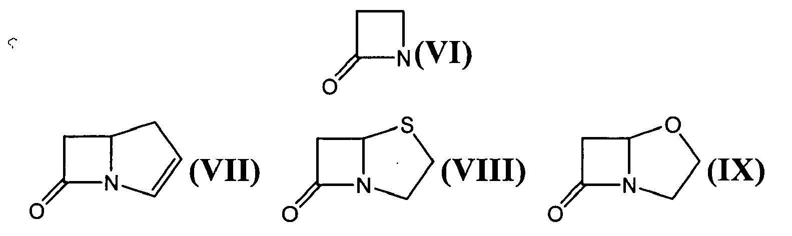

The chloramphenicol group antibiotics of the present invention refer to synthetic, semi-synthetic or natural antibiotics or derivatives thereof having a structure similar with chloramphenicol (X). For example, they are, but not limited to chloramphenicol, chloramphenicol succinate, thiamphenicol, etc.

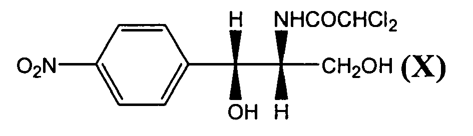

The lincomycin group antibiotics of the present invention refer to antibiotics having a structure of lincomycin (XI) or analogs or derivatives thereof. For example, they are, but not limited to lincomycin, chlindamycin, etc.

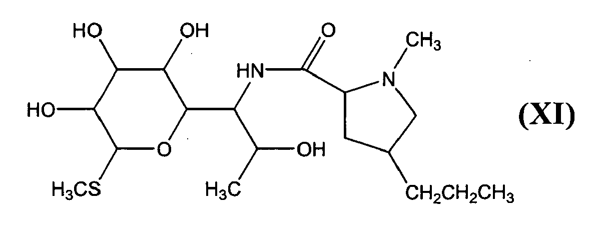

The macrolide group antibiotics of the present invention refer to, but are not limited to macrolides, such as erythromycin, spiramycin (XII), roxithromycin, azithromycin, etc., and derivatives thereof, such as salts, esters, etc.

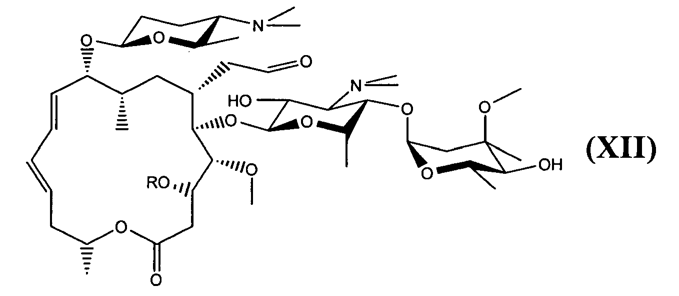

The quinolones of the present invention are synthetic chemotherapeutic drugs and do not belong to antibiotic field, but for conciseness, the present invention considers them as antibiotics according to their drug actions, and does not distinguish them in the description. Quinolones include, but are not limited to norfloxacin, ofloxacin and ciprofloxacin having nalidixic acid parent cores (XIII); enoxacin having quinoline carboxylic acid parent cores (XIV); and pipemidic acid having pyridinopyrimidine carboxylic acid parent cores (XV).

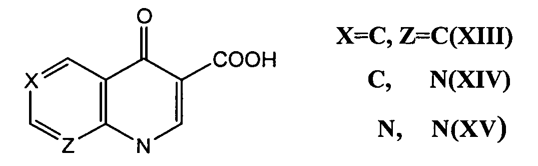

The tetracyclines of the present invention refer to natural, semi-synthetic or synthetic antibiotics that are produced by actinomycete, etc. and have phenanthrene basic framework. For example, they are, but not limited to tetracycline (XVI), aureomycin, terramycin, etc.

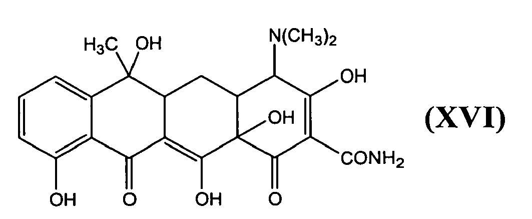

Bacteria that can be induced by antibiotics to generate CGCs include, but are not limited to enteric gram-negative bacillus, including pathogenic bacteria and indigenous flora, anaerobic and amphoteric bacteria.

Although the present invention does not stand on any known theory, the mechanism that the combination of N-acetyl-D-glucosamine and antibiotics in the present invention enhances drug action is deemed as follows:
Antibiotics bring about effects by reacting with bacteria. When bacteria convert into CGCs, they do not react with antibiotics, so that antibiotics are ineffective. N-acetyl-D-glucosamine reverts the cryptic growth cells to bacteria in vegetative form that are sensitive to antibiotics, so that the drug action of antibiotics is enhanced.

When the compound preparations of the present invention comprise N-acetyl-D-glucosamine and antibiotics simultaneously, the dosages and proportions of N-acetyl-D-glucosamine and antibiotics depend on the kind of antibiotics. Specifically, since N-acetyl-D-glucosamine has antibiotic synergetic effect, the dosage of antibiotic may be its conventional dosage of less than its conventional dosage. For example, the dosage of antibiotic may be reduced to 50% or less of its conventional dosage. N-acetyl-D-glucosamine is nontoxic, so that its dosage is not specifically limited. For example, the daily dosage of N-acetyl-D-glucosamine may range from 100mg to 10g. Specifically:

In case that N-acetyl-D-glucosamine combines with aminoglycoside antibiotics, the ratio of N-acetyl-D-glucosamine to aminoglycoside antibiotics ranges from 1:1.6 to 1:5. For example, compound streptomycin injection may have an adult dose of lg/day by intravenous drop or intramuscular injection. Compound kanamycin injection may have a dose of 300mg/day, once per 8 hours, by intramuscular injection. Compound gentamycin injection may have a dose of 90-300mg/day, once per 8 hours, by intramuscular injection or intravenous drop. The above doses are based on antibiotics (similarly hereinafter).

When N-acetyl-D-glucosamine combines with macrolide group antibiotics, the ratio of N-acetyl-D-glucosamine to macrolide group antibiotics ranges from 1:5 to 1:30. For example, compound spiramycin is formulated according to the ratio to form capsules, and has a dose of 2-3g/day, once per 8 hours, by oral administration.

When N-acetyl-D-glucosamine combines with quinolones, the ratio of N-acetyl-D-glucosamine to quinolones ranges from 1:1 to 1:15. For example, compound norfloxacin is formulated to form capsules, and has a dose of 1200mg/day, once per 8 hours, by oral administration. Compound ciprofloxacin capsule has a dose of 1200mg/day, once per 8 hours, by oral administration; or compound ciprofloxacin injection has a dose of 200mg/day, once or twice per day, by intravenous drop.

When N-acetyl-D-glucosamine combines with lincomycin group antibiotics, the ratio of N-acetyl-D-glucosamine to lincomycin group antibiotics ranges from 1:2.5 to 1:10. For example, capsule formulated with powders may have a dose of 0.6-1.8g/day; or it is formulated to form injection for intravenous drop.

When N-acetyl-D-glucosamine combines with chloramphenicol group antibiotics, the ratio of N-acetyl-D-glucosamine to chloramphenicol group antibiotics ranges from 1:2.5 to 1:10. Sugar coated tablet or syrup formulated with powders may have a dose of 25-50mg/kg by oral administration; or it is formulated as an injection having a dose of 1-2g/day, 2-4 times per day, for intramuscular injection or intravenous drop.

When N-acetyl-D-glucosamine combines with tetracyclines, the ratio of N-acetyl-D-glucosamine to tetracyclines ranges from 1:1 to 1:30. Capsule or sugar coated tablet may have a dose of 1-2g/day by oral administration; or it is formulated as an injection having a dose of 1-1.5g/day for intravenous drop.

When N-acetyl-D-glucosamine combines with cephalosporin group antibiotics, the ratio of N-acetyl-D-glucosamine to cephalosporin group antibiotics ranges from 1:2.6 to 1:5. For example, an injection obtained by mixing N-acetyl-D-glucosamine and cefuroxime may have a dose of 1.5-6g/day, twice per day, by intravenous drop or intramuscular injection; and an injection obtained by mixing N-acetyl-D-glucosamine and cefotaxime may have a dose of 2g/day, twice per day, by intravenous drop or intramuscular injection.

When N-acetyl-D-glucosamine combines with penicillin group antibiotics, the ratio of N-acetyl-D-glucosamine to penicillin group antibiotics ranges from 1:1 to 1:30. For example, N-acetyl-D-glucosamine is mixed with ampicillin to form a capsule having a dose of 50-100mg/kg/day for oral administration, or to form an injection having a dose of 100-200mg/kg/day for intravenous drop or intramuscular injection; and N-acetyl-D-glucosamine is mixed with carbenicillin to form an injection having a dose of 4-8g/day, 4 times per day, for intravenous drop or intramuscular injection.

When N-acetyl-D-glucosamine combines with other β-lactam antibiotics, the ratio of N-acetyl-D-glucosamine to other β-lactam antibiotics ranges from 1:8 to 1:50. For example, N-acetyl-D-glucosamine is mixed with cefoxitin to form an injection having a dose of 8-10g/day for intravenous drop or intramuscular injection; and N-acetyl-D-glucosamine is mixed with ampicillin-sulbactam to form an injection having a dose of 1.5-6g/day, 2-3 times per day, for intravenous drop or intramuscular injection.

It should be understood that when N-acetyl-D-glucosamine combines with an antibiotic, they are formulated to form a compound preparation so that they are used simultaneously, or are formulated separately and are administered simultaneously or one after the other. For example, the antibiotic is administered firstly, and then N-acetyl-D-glucosamine is administered after a while; or N-acetyl-D-glucosamine is administered firstly, and then the antibiotic is administered. There is no restriction on this aspect.

The following experimental examples are to illustrate the effects of combinations of N-acetyl-D-glucosamine and antibiotics in the present invention for combating CGC and for prevention and treatment of dysbacteriosis in intestinal tract, irritable bowel syndrome, etc.

The antibiotics used hereinafter were all commercial products, and were purchased from the Pharmacy of Xinan Hospital, The Third Military Medical University.

### Test Example 1

Tests of inducing E. coli CGC by using compound antibacterial agents comprising aminoglycoside and N-acetyl-D-glucosamine in different ratios

E. coli (No. 33310, purchased from Chengdu Institute of Biological Products, the Ministry of Public Health) was used in the tests. Grid design was performed by using antibiotic with different concentrations and N-acetyl-D-glucosamine having an amount of from 10mg to 300mg. Streak plates were conducted separately, and K-B method was employed for drugs. Colonies at marginal part of drug inhibition zone were picked and were observed under microscope. If cell length was more than 50*µ*m and cell number was more than 5 in each visual field, it was deemed as CGC positive and was marked with "+"; while if cell length was not greater than 50*µ*m and cell number was within the range from 0 to 4 in each visual field, it was deemed as CGC negative and was marked with "-". At this time, the ratio of two substances was called "effective ratio". The results are shown in Table 1-1 to Table 1-9.

**Table 1-1 Aminoglycoside was selected from kanamycin and gentamycin and had an amount of from 50mg to 500mg.**

| Aminoglycosides (mg) | 50 | 100 | 150 | 200 | 300 | 400 | 500 |
|---|---|---|---|---|---|---|---|
| Compound (I) (mg) | | | | | | | |
| 10 | - | + | + | + | + | + | + |
| 50 | - | - | + | + | + | + | + |
| 100 | - | - | - | + | + | + | + |
| 150 | - | - | - | - | + | + | + |
| 200 | - | - | - | - | - | + | + |
| 250 | - | - | - | - | - | - | + |
| 300 | - | - | - | - | - | - | - |

Conclusion: the effective ratio of N-acetyl-D-glucosamine to aminoglycoside in the compound antibacterial agent for preventing the formation of E. coli CGC is from 1:1.6 to 1:5.

**Table 1-2 The macrolide group antibiotic was selected from spiramycin and had an amount of from 300mg to 1500mg.**

| Macrolides(mg) | 300 | 500 | 700 | 900 | 1100 | 1300 | 1500 |
|---|---|---|---|---|---|---|---|
| Compound (I) (mg) | | | | | | | |
| 10 | - | + | + | + | + | + | + |
| 50 | - | - | + | + | + | + | + |
| 100 | - | - | - | + | + | + | + |
| 150 | - | - | - | - | + | + | + |
| 200 | - | - | - | - | - | + | + |
| 250 | - | - | - | - | - | - | + |
| 300 | - | - | - | - | - | - | - |

Conclusion: the effective ratio of N-acetyl-D-glucosamine to macrolide in the compound antibacterial agent for preventing the formation of E. coli CGC is from 1:5 to 1:30.

**Table 1-3 The quinoline was selected from ciprofloxacin and norfloxacin and had an amount of from 150mg to 600mg.**

| Quinolines (mg) | 150 | 200 | 300 | 400 | 500 | 550 | 600 |
|---|---|---|---|---|---|---|---|
| Compound (I) (mg) | | | | | | | |
| 10 | - | + | + | + | + | + | + |
| 50 | - | - | + | + | + | + | + |
| 100 | - | - | - | + | + | + | + |
| 150 | - | - | - | - | + | + | + |
| 200 | - | - | - | - | - | + | + |
| 250 | - | - | - | - | - | - | + |
| 300 | - | - | - | - | - | - | - |

Conclusion: the effective ratio of N-acetyl-D-glucosamine to quinolines in the compound antibacterial agent for preventing the formation of E. coli CGC was from 1:2 to 1:15.

**Table 1-4 The lincomycin group antibiotics were selected from lincomycin, and had an amount of from 100mg to 700mg.**

| Lincomycin (mg) | 100 | 200 | 300 | 400 | 500 | 600 | 700 |
|---|---|---|---|---|---|---|---|
| Compound (I) (mg) | | | | | | | |
| 10 | - | + | + | + | + | + | + |
| 50 | - | - | + | + | + | + | + |
| 100 | - | - | - | + | + | + | + |
| 150 | - | - | - | - | + | + | + |
| 200 | - | - | - | - | - | + | + |
| 250 | - | - | - | - | - | - | + |
| 300 | - | - | - | - | - | - | - |

Conclusion: the effective ratio of N-acetyl-D-glucosamine to lincomycin in the compound antibacterial agent for preventing the formation of E. coli CGC was from 1:2.5 to 1:10.

**Table 1-5 Chloramphenicol had an amount of from 100mg to 700mg.**

| Chloramphenical (mg) | 100 | 200 | 300 | 400 | 500 | 600 | 700 |
|---|---|---|---|---|---|---|---|
| Compound (I) (mg) | | | | | | | |
| 10 | - | + | + | + | + | + | + |
| 50 | - | - | + | + | + | + | + |
| 100 | - | - | - | + | + | + | + |
| 150 | - | - | - | - | + | + | + |
| 200 | - | - | - | - | - | + | + |
| 250. | - | - | - | - | - | - | + |
| 300 | - | - | - | - | - | - | - |

Conclusion: the effective ratio of N-acetyl-D-glucosamine to chloramphenical in the compound antibacterial agent for preventing the formation of E. coli CGC was from 1:2.5 to 1:10.

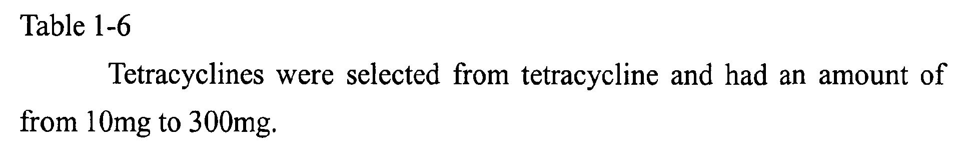

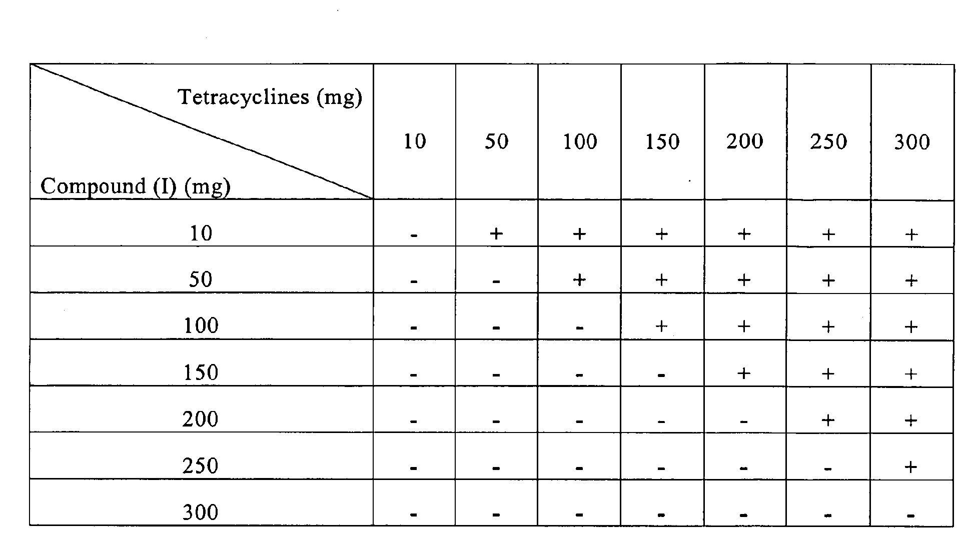

Conclusion: the effective ratio of N-acetyl-D-glucosamine to tetracyclines in the compound antibacterial agent for preventing the formation of E. coli CGC was from 1:1 to 1:30.

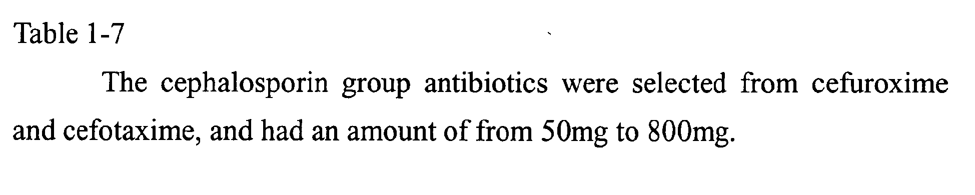

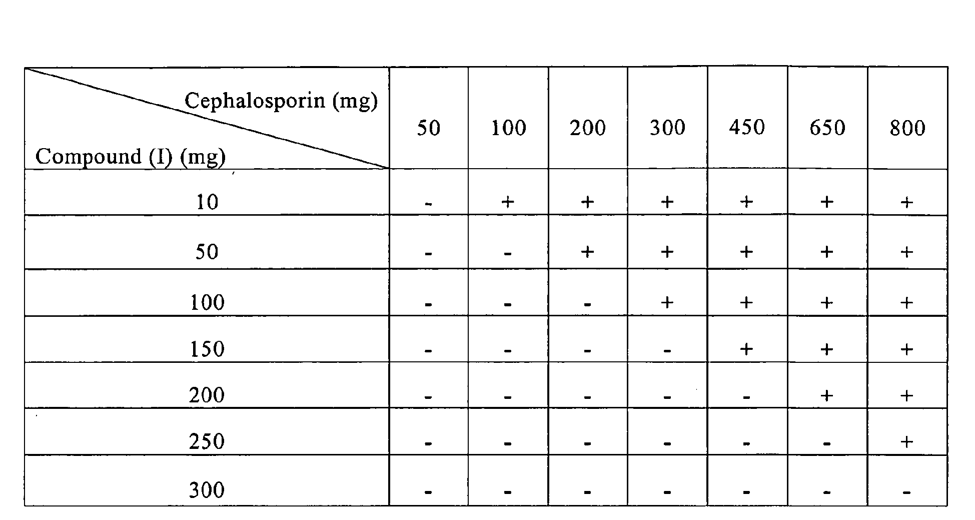

Conclusion: the effective ratio of N-acetyl-D-glucosamine to cephalosporin group antibiotics in the compound antibacterial agent for preventing the formation of E. coli CGC was from 1:2.6 to 1:5.

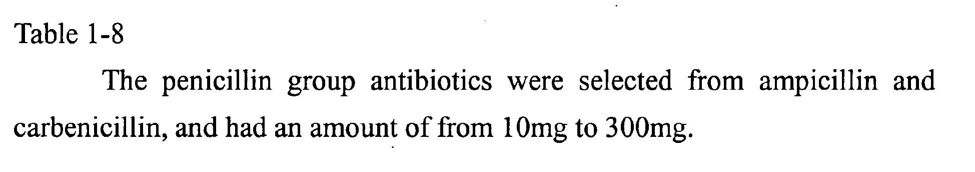

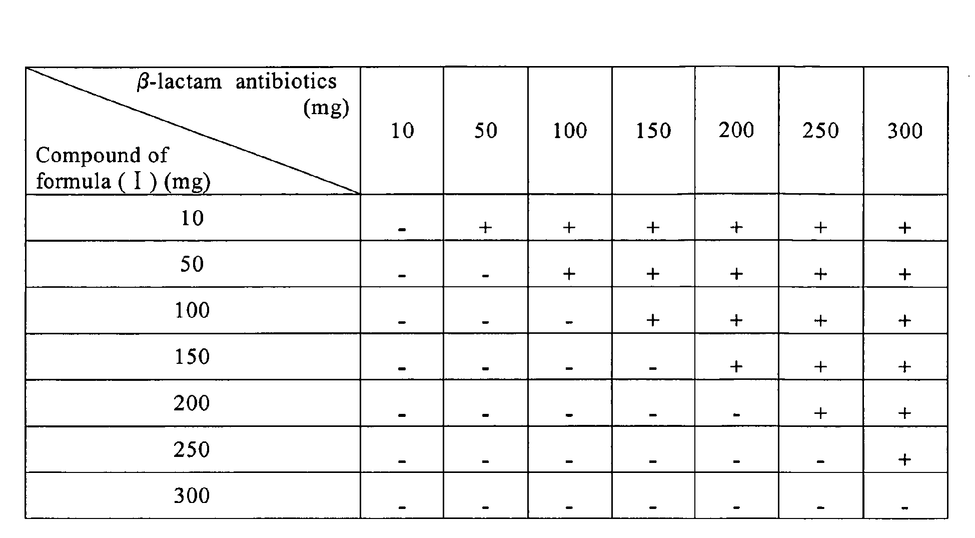

Conclusion: the effective ratio of N-acetyl-D-glucosamine to β-lactam antibiotics in the compound antibacterial agent for preventing the formation of E. coli CGC was from 1:1 to 1:30.

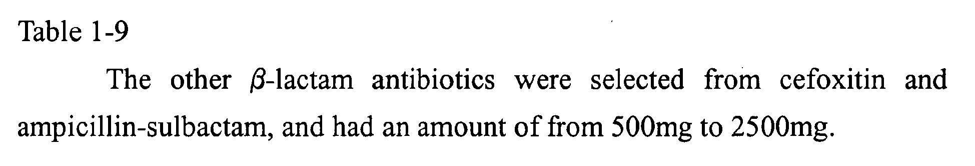

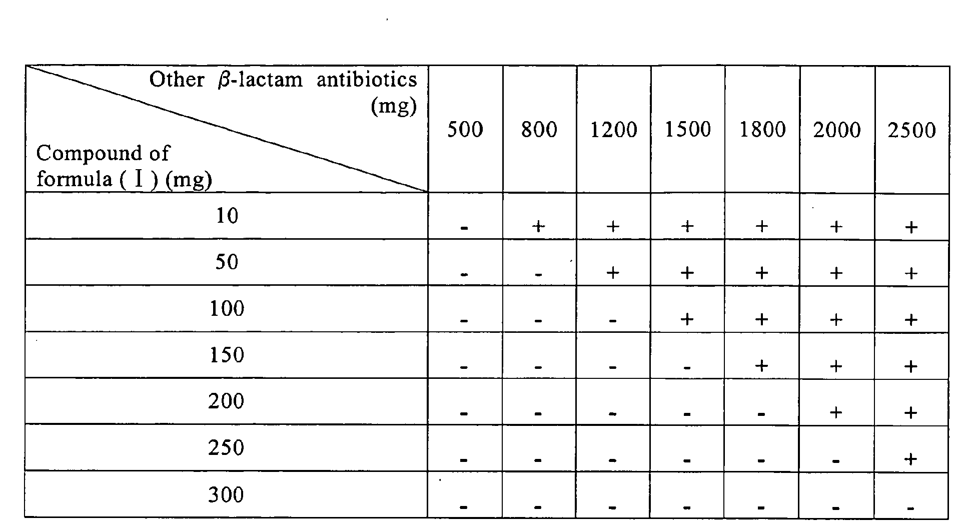

Conclusion: the effective ratio of N-acetyl-D-glucosamine to other *β*-lactam antibiotics in the compound antibacterial agent for preventing the formation of E. coli CGC was from 1:8 to 1:50.

### Test Example 2

Effect tests of compound preparation comprising N-acetyl-D-glucosamine and antibiotics for preventing the formation of bacterial CGC in vitro.

The tests were carried out in vitro, wherein gram-negative amphimicrobes, gram-negative anaerobes and gram-positive aerobes were separately coated on streak plates, test drugs and control drugs were separately dissolved with same volume of sterile water for injection, 10*µ*l of each drug was used for preparing drug-sensitive paper, K-B method was employed in the tests of drugs, and the inhibition situations of CGC formation were observed. The results of the tests were shown in Table 2-1 to Table 2-9.
Table 2-1 The compound injection was prepared by using 10mg of N-acetyl-D-glucosamine and 30mg of kanamycin (an aminoglycoside) of Example 1, and the control drug was kanamycin with the same dose.

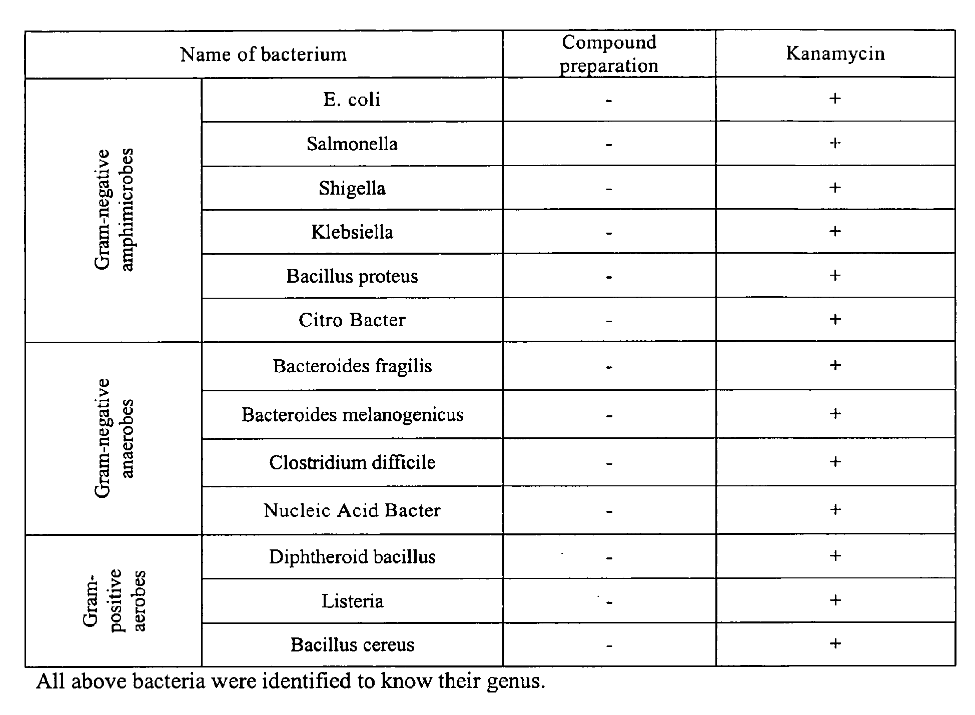

Conclusion: in comparison with aminoglycoside alone, the compound antibacterial agent of the combination of N-acetyl-D-glucosamine and aminoglycoside effectively prevented common bacteria in digestive tract from converting into CGC in vitro.
Table 2-2 The compound preparation of N-acetyl-D-glucosamine and macrolide group antibiotic of Example 1 was prepared by mixing powders (100mg of N-acetyl-D-glucosamine, 900mg of spiramycin powder), and the control drug was 900mg of spiramycin.

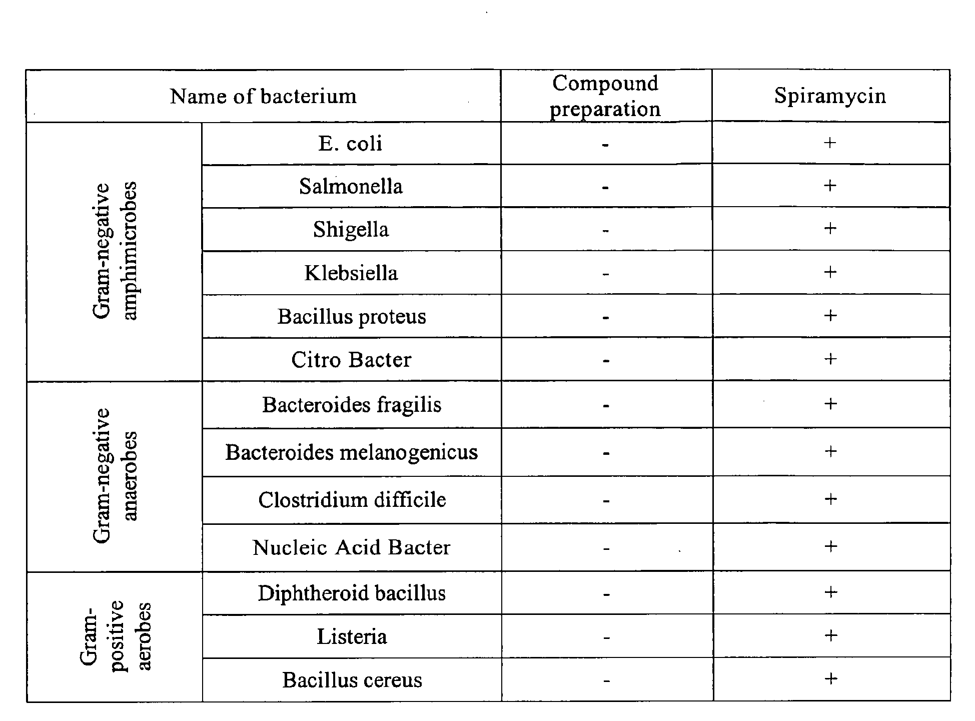

Conclusion: in comparison with macrolide group antibiotic alone, the compound antibacterial agent of the combination of N-acetyl-D-glucosamine and macrolide group antibiotic effectively prevented common bacteria in digestive tract from converting into CGC in vitro.

### Table 2-3

The compound preparation of N-acetyl-D-glucosamine and quinoline of Example 1 was prepared by mixing 100mg of N-acetyl-D-glucosamine and 400mg of norfloxacin, and the control drug was norfloxacin with the same dose.

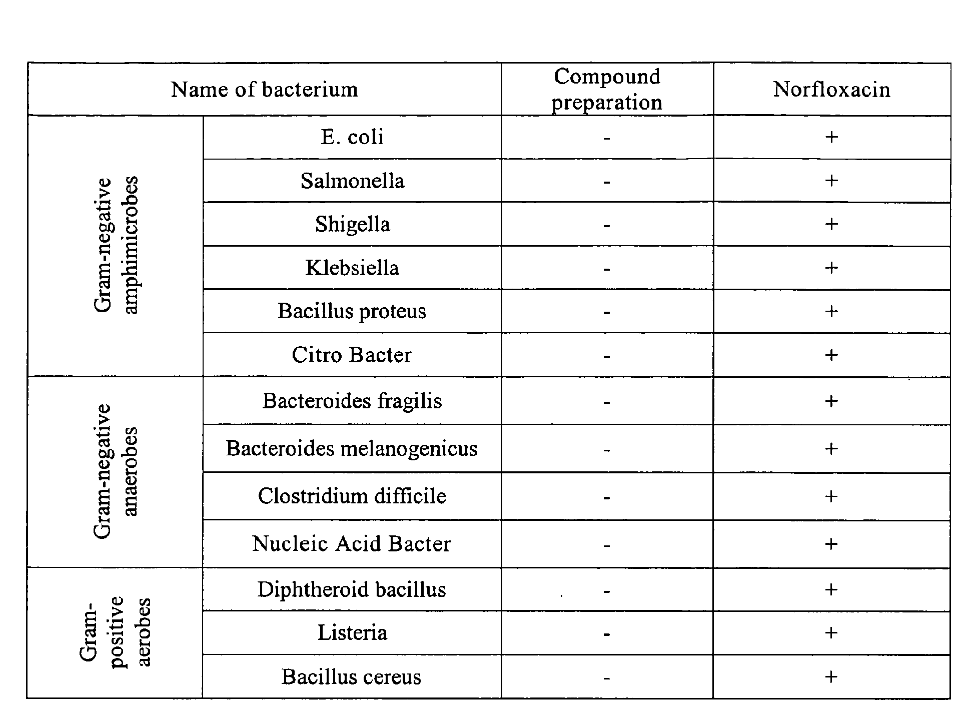

Conclusion: in comparison with quinoline alone, the compound antibacterial agent of the combination of N-acetyl-D-glucosamine and quinoline effectively prevented common bacteria in digestive tract from converting into CGC in vitro.
Table 2-4 The compound preparation of N-acetyl-D-glucosamine (100mg) and lincomycin (400mg) of Example 1 was prepared, and the control drug was lincomycin (400mg).

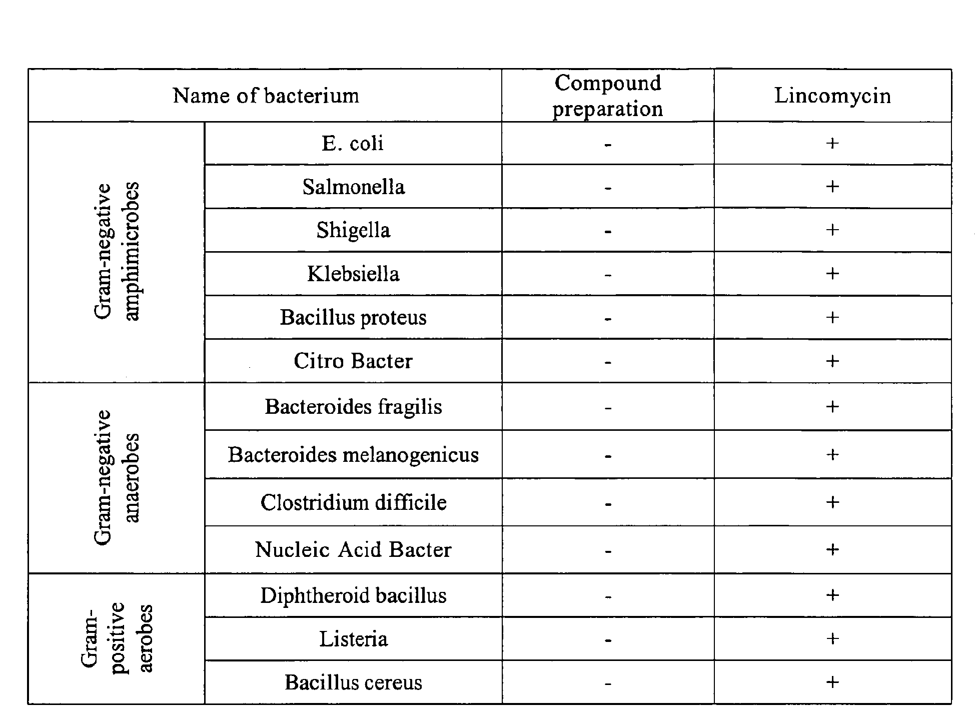

Conclusion: in comparison with lincomycin alone, the compound antibacterial agent of the combination of N-acetyl-D-glucosamine and lincomycin effectively prevented common bacteria in digestive tract from converting into CGC in vitro.
Table 2-5 The compound preparation of N-acetyl-D-glucosamine (100mg) and chloramphenicol (400mg) of Example 1 was prepared, and the control drug was chloramphenicol (400mg).

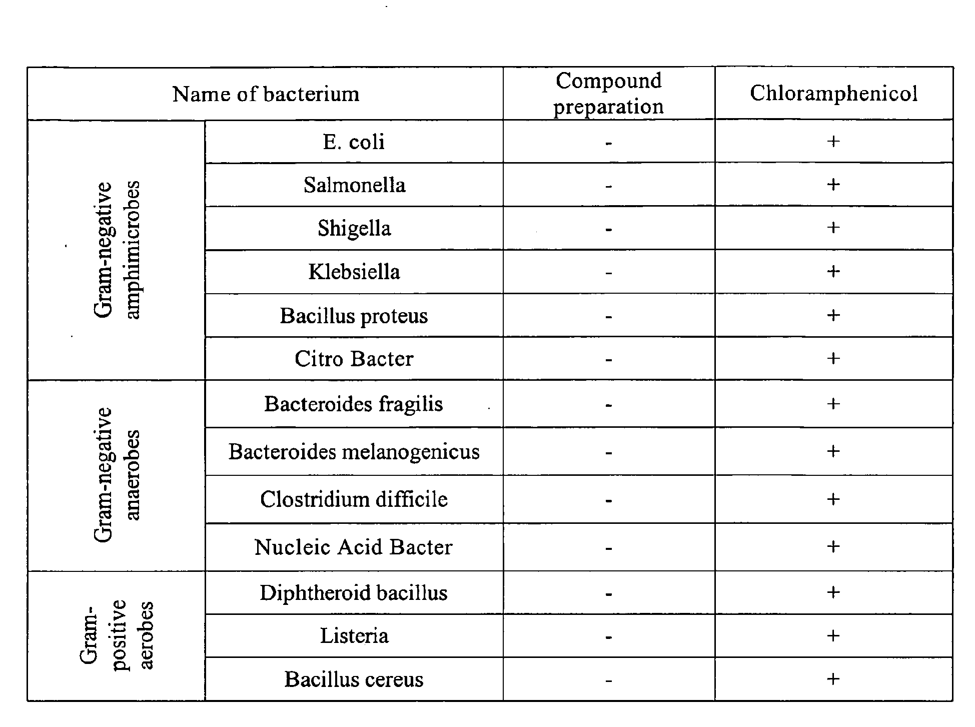

Conclusion: in comparison with chloramphenicol alone, the compound antibacterial agent of the combination of N-acetyl-D-glucosamine and chloramphenicol effectively prevented common bacteria in digestive tract from converting into CGC in vitro.
Table 2-6 The compound preparation of N-acetyl-D-glucosamine (50mg) and tetracycline (150mg) of Example 1 was prepared, and the control drug was tetracycline (150mg).

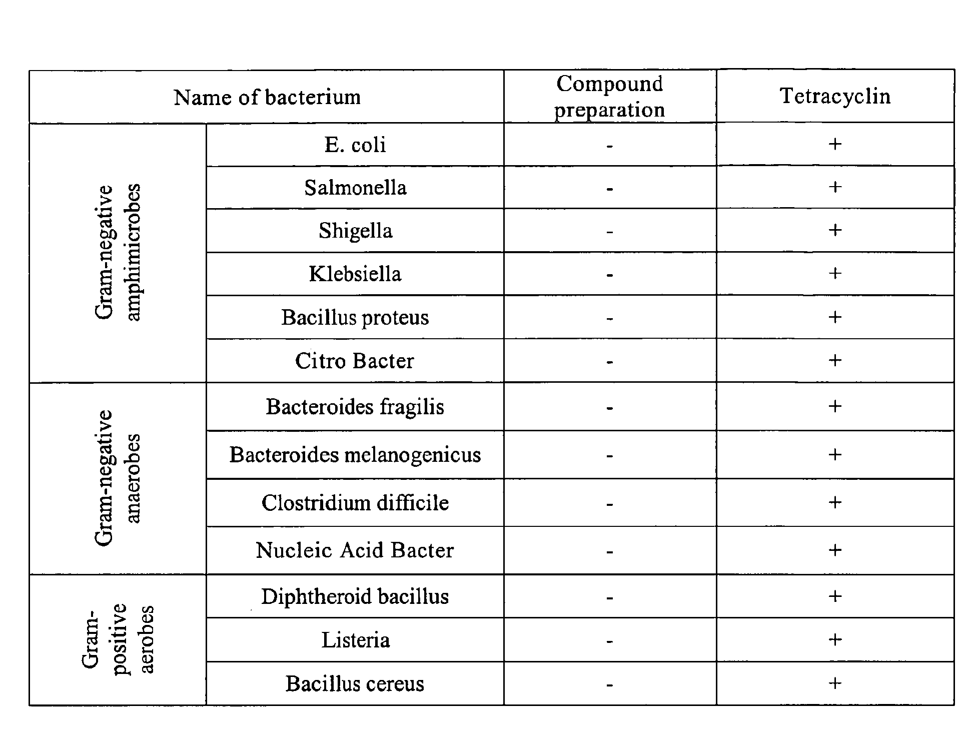

Conclusion: in comparison with tetracycline alone, the compound antibacterial agent of the combination of N-acetyl-D-glucosamine and tetracycline effectively prevented common bacteria in digestive tract from converting into CGC in vitro.
Table 2-7 The compound preparation of N-acetyl-D-glucosamine and cephalosporin group antibiotics of Example 1 was prepared by mixing 100mg of N-acetyl-D-glucosamine and 300mg of cefuroxime, and the control drug was 300mg of cefuroxime.

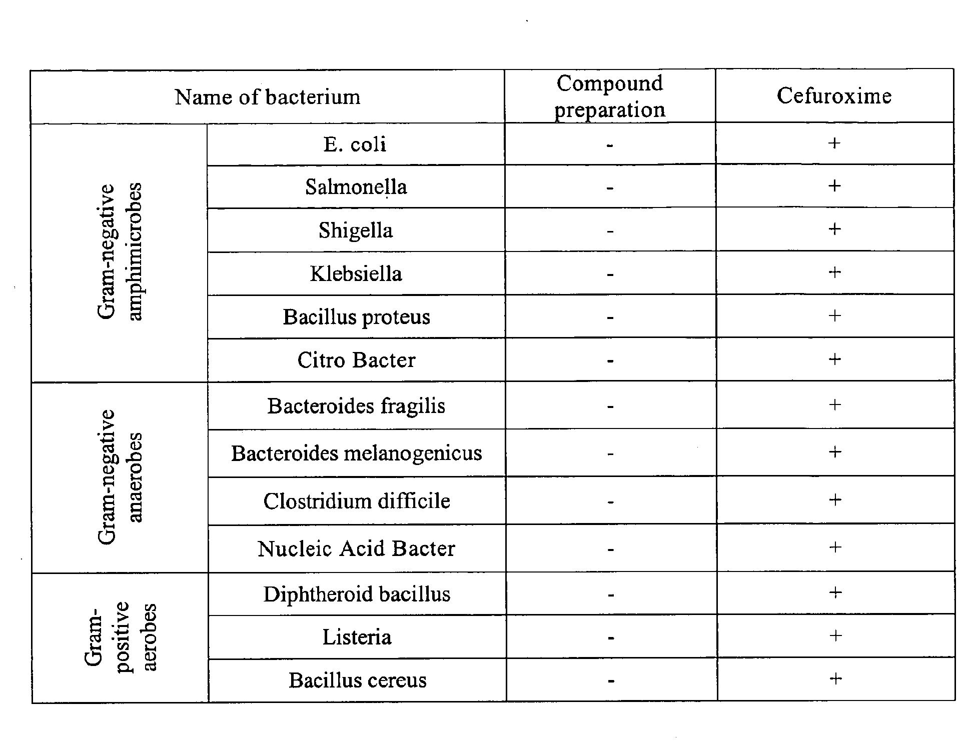

Conclusion: in comparison with cephalosporin group antibiotics alone, the compound antibacterial agent of the combination of N-acetyl-D-glucosamine and cephalosporin group antibiotics effectively prevented common bacteria in digestive tract from converting into CGC in vitro.
Table 2-8 The compound preparation of N-acetyl-D-glucosamine and penicillin group antibiotics of Example 1 was prepared by mixing 150mg of N-acetyl-D-glucosamine and 150mg of ampicillin, and the control drug was ampicillin.

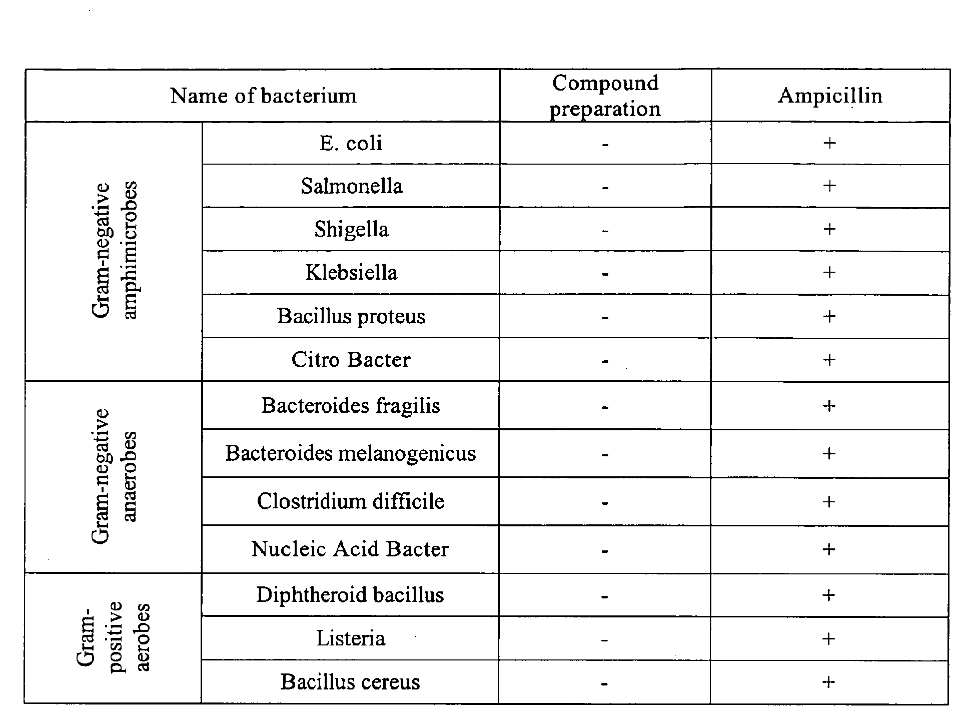

Conclusion: in comparison with β-lactam antibiotics alone, the compound antibacterial agent of the combination of N-acetyl-D-glucosamine and β-lactam antibiotics effectively prevented common bacteria in digestive tract from converting into CGC in vitro.
Table 2-9 The compound preparation of N-acetyl-D-glucosamine and other β-lactam antibiotics of Example 1 was prepared by mixing 100mg of N-acetyl-D-glucosamine and 1500mg of ampicillin-sulbactam, and the control drug was ampicillin-sulbactam.

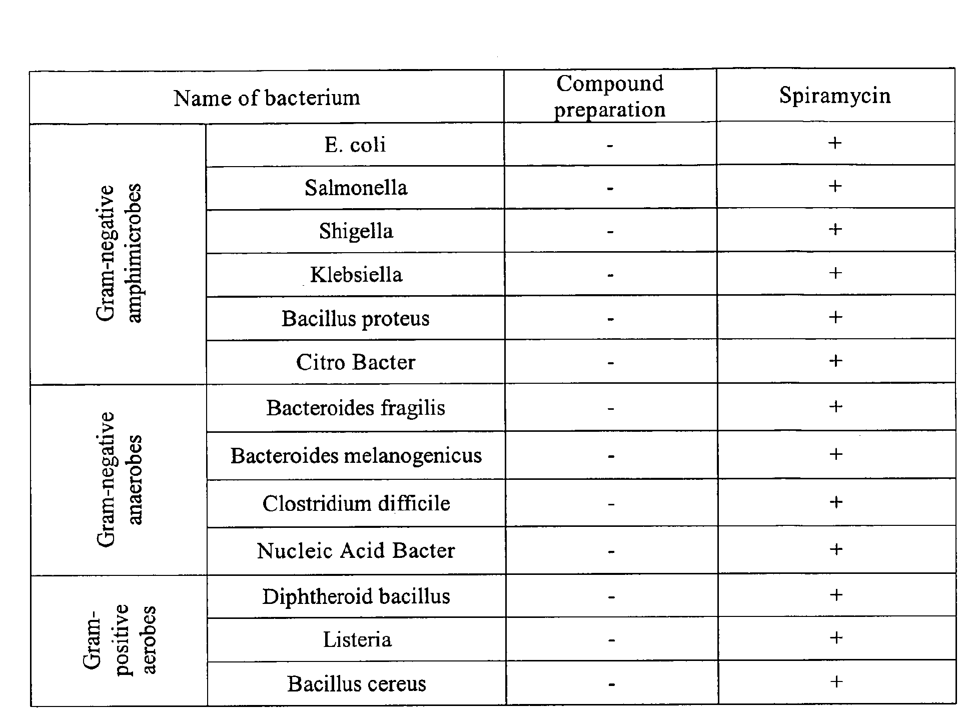

Conclusion: in comparison with other β-lactam antibiotics alone, the compound antibacterial agent of the combination of N-acetyl-D-glucosamine and other β-lactam antibiotics effectively prevented common bacteria in digestive tract from converting into CGC in vitro. Test Example 3

Effect tests of compound antibacterial preparation comprising N-acetyl-D-glucosamine and antibiotics for preventing the formation of CGC in vivo

Wistar rats were used in the tests. In vivo tests of using compound preparation comprising N-acetyl-D-glucosamine and antibiotics with effective proportions to treat rats infected and not infected by Bacillus typhi murium were conducted by experimental observation (the proportions of drugs were given in the following examples, and the dosage forms were those abovementioned). Equivalent amount of antibiotics without N-acetyl-D-glucosamine was used as control. The dose for rats was 6.5 times as high as the dose for human (dosage for per kilogram, as abovementioned). Rats were grouped randomly, and each group had 15 rats.

Rats were intramuscularly or orally administered with effective dose for a consecutive week. Two stool CGC detections were conducted per day, and intestinal mucosa CGC colonization detection was conducted on the 7th day in order to determine the coincidence relation with the stool CGCs. It is positive if stool has CGC and intestinal mucosa has CGC colonization; on the contrary, it is negative. The expression manner is that the total number of animals is denominator and the number of positive animals is numerator. The results are as follows.

### Test Example 3-1: Aminoglycoside antibiotics

Drug: N-acetyl-D-glucosamine (100mg) + kanamycin (200mg)

Conclusion: the positive rates of stool CGC and intestinal mucosa CGC of rats infected or not by Bacillus typhi murium which were administered with an effective dose of a compound antibacterial agent of N-acetyl-D-glucosamine and aminoglycosides were 0 (0/15), while all CGC positive rates of infected rats and not infected rats which were administered with only kanamycin were 100% (15/15). The stool CGC positive results were coincident with the intestinal mucosa CGC colonization positive results. This indicates that the compound antibacterial preparation of N-acetyl-D-glucosamine and aminoglycosides is capable of effectively preventing the formation of CGC in vivo.

### Test Example 3-2: Macrolide group antibiotic

Drug: N-acetyl-D-glucosamine (100mg) + spiramycin (900mg)

Conclusion: the positive rates of stool CGC and intestinal mucosa CGC of rats infected or not by Bacillus typhi murium which were administered with an effective dose of a compound antibacterial agent of N-acetyl-D-glucosamine and macrolide group antibiotic were 0 (0/15), while all CGC positive rates of infected rats and not infected rats which were administered with only spiramycin were 100% (15/15). The stool CGC positive results were coincident with the intestinal mucosa CGC colonization positive results. This indicates that the compound antibacterial preparation of N-acetyl-D-glucosamine and macrolide group antibiotic is capable of effectively preventing the formation of CGC in vivo.

### Test Example 3-3: Quinolines

### Drug: N-acetyl-D-glucosamine (100mg) + ciprofloxacin (500mg)

Conclusion: the positive rates of stool CGC and intestinal mucosa CGC of rats infected or not by Bacillus typhi murium which were administered with an effective dose of a compound antibacterial agent of N-acetyl-D-glucosamine and quinoline were 0 (0/15), while all CGC positive rates of infected rats and not infected rats which were administered with only ciprofloxacin were 100% (15/15). The stool CGC positive results were coincident with the intestinal mucosa CGC colonization positive results. This indicates that the compound antibacterial preparation of N-acetyl-D-glucosamine and quinolines is capable of effectively preventing the formation of CGC in vivo.

### Test Example 3-4: Lincomycin group antibiotics

### Drug: N-acetyl-D-glucosamine (100mg) + lincomycin (400mg)

Conclusion: the positive rates of stool CGC and intestinal mucosa CGC of rats infected or not by Bacillus typhi murium which were administered with an effective dose of a compound antibacterial agent of N-acetyl-D-glucosamine and lincomycin group antibiotics were 0 (0/15), while all CGC positive rates of infected rats and not infected rats which were administered with only lincomycin were 100% (15/15). The stool CGC positive results were coincident with the intestinal mucosa CGC colonization positive results. This indicates that the compound antibacterial preparation of N-acetyl-D-glucosamine and lincomycin group antibiotics is capable of effectively preventing the formation of CGC in vivo.

### Test Example 3-5: Chloramphenicol group antibiotics

### Drug: N-acetyl-D-glucosamine (100mg) + chloramphenical (400mg)

Conclusion: the positive rates of stool CGC and intestinal mucosa CGC of rats infected or not by Bacillus typhi murium which were administered with an effective dose of a compound antibacterial agent of N-acetyl-D-glucosamine and chloramphenicol group antibiotics were 0 (0/15), while all CGC positive rates of infected rats and not infected rats which were administered with only chloramphenicol were 100% (15/15). The stool CGC positive results were coincident with the intestinal mucosa CGC colonization positive results. This indicates that the compound antibacterial preparation of N-acetyl-D-glucosamine and chloramphenicol group antibiotics is capable of effectively preventing the formation of CGC in vivo.

### Test Example 3-6: Tetracycline group antibiotics

### Drug: N-acetyl-D-glucosamine (150mg) + tetracycline (150mg)

Conclusion: the positive rates of stool CGC and intestinal mucosa CGC of rats infected or not by Bacillus typhi murium which were administered with an effective dose of a compound antibacterial agent of N-acetyl-D-glucosamine and tetracycline group antibiotics were 0 (0/15), while all CGC positive rates of infected rats and not infected rats which were administered with only tetracycline were 100% (15/15). The stool CGC positive results were coincident with the intestinal mucosa CGC colonization positive results. This indicates that the compound antibacterial preparation of N-acetyl-D-glucosamine and tetracycline group antibiotics is capable of effectively preventing the formation of CGC in vivo.

### Test Example 3-7: Cephalosporin group antibiotics

### Drug: N-acetyl-D-glucosamine (100mg) + cefuroxime (300mg)

Conclusion: the positive rates of stool CGC and intestinal mucosa CGC of rats infected or not by Bacillus typhi murium which were administered with an effective dose of a compound antibacterial agent of N-acetyl-D-glucosamine and cephalosporin group antibiotics were 0 (0/15), while all CGC positive rates of infected rats and not infected rats which were administered with only cefuroxime were 100% (15/15). The stool CGC positive results were coincident with the intestinal mucosa CGC colonization positive results. This indicates that the compound antibacterial preparation of N-acetyl-D-glucosamine and cephalosporin group antibiotics is capable of effectively preventing the formation of CGC in vivo.

### Test Example 3-8: Penicillin group antibiotics

### Drug: N-acetyl-D-glucosamine (100mg) + ampicillin (200mg)

Conclusion: the positive rates of stool CGC and intestinal mucosa CGC of rats infected or not by Bacillus typhi murium which were administered with an effective dose of a compound antibacterial agent of N-acetyl-D-glucosamine and penicillin group antibiotics were 0 (0/15), while all CGC positive rates of infected rats and not infected rats which were administered with only ampicillin were 100% (15/15). The stool CGC positive results were coincident with the intestinal mucosa CGC colonization positive results. This indicates that the compound antibacterial preparation of N-acetyl-D-glucosamine and penicillin group antibiotics is capable of effectively preventing the formation of CGC in vivo.

### Test Example 3-9: Other β-lactam antibiotics

### Drug: N-acetyl-D-glucosamine (100mg) + cefoxitin (150mg)

Conclusion: the positive rates of stool CGC and intestinal mucosa CGC of rats infected or not by Bacillus typhi murium which were administered with an effective dose of a compound antibacterial agent of N-acetyl-D-glucosamine and other β-lactam antibiotics were 0 (0/15), while all CGC positive rates of infected rats and not infected rats which were administered with only cefoxitin were 100% (15/15). The stool CGC positive results were coincident with the intestinal mucosa CGC colonization positive results. This indicates that the compound antibacterial preparation of N-acetyl-D-glucosamine and other β-lactam antibiotics is capable of effectively preventing the formation of CGC in vivo.

### Test Example 4

Experimental observation of effects of compound preparation of N-acetyl-D-glucosamine and antibiotics for preventing dysbacteriosis caused by bacterial CGC

Each control group had 15 rats and was administered with only kanamycin, gentamycin, spiramycin, ciprofloxacin, norfloxacin, lincomycin, chloramphenicol, tetracycline, cefuroxime, cefotaxime, ampicillin, carbenicillin, cefoxitin or ampicillin-sulbactam in an effective dosage respectively, twice per day, for consecutive 15 days, and the intestinal flora results were detected. Test groups were separately administered with an effective dosage of compound preparation of N-acetyl-D-glucosamine and kanamycin, gentamycin, spiramycin, ciprofloxacin, norfloxacin, lincomycin, chloramphenicol, tetracycline, cefuroxime, cefotaxime, ampicillin, carbenicillin, cefoxitin or ampicillin-sulbactam for consecutive 15 days, and the intestinal flora results were detected was well. The results showed that the kinds of indigenous flora in intestinal tract of rats in control groups were reduced from 12 to 5, the ratio of gram-positive bacillus to negative bacteria was changed, and the stool water content increased from average 45% to 60% (diarrhea), while these did not appear in the rats of test groups. Conclusion: the compound preparations do not cause dysbacteriosis so that the occurrence of dysbacteriosis is avoided.

### Test Example 5

### Effect tests of compound antibacterial preparation of N-acetyl-D-glucosamine and antibiotics for effectively preventing irritable bowel syndrome (IBS) caused by bacterial CGC

60 rats were randomly divided into test groups and control group, and each group had 30 rats. The rats of test groups were administered with an effective dosage of N-acetyl-D-glucosamine and antibiotics, while the rats of control group were administered with only an effective dosage of antibiotics. The rats of test groups were administered with an effective dosage of a compound antibacterial preparation of N-acetyl-D-glucosamine and aminoglycoside by intramuscular injection for consecutive 10 days. The rat stools were observed during the period of administration and within 1 week after drug withdrawal in order to determine whether CGC existed. CGC was not detected in rat stools after one week from drug withdrawal. On this basis, the rats were subjected to stimulations such as electric stimulation, intragastric administration with fructus zanthoxyli water, cold, constraint etc. in order to induce IBS, and the incidence rates of IBS of two groups were observed.

### Test Example 5-1: Aminoglycoside antibiotics

Drug: N-acetyl-D-glucosamine + kanamycin, control: kanamycin

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and aminoglycosides is capable of effectively preventing the occurrence of irritable bowel syndrome.

### Test Example 5-2: Macrolide group antibiotic

### Drug: N-acetyl-D-glucosamine + spiramycin, control: spiramycin

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and macrolide group antibiotic is capable of effectively preventing the occurrence of irritable bowel syndrome.

### Test Example 5-3: Quinolines

### Drug: N-acetyl-D-glucosamine + ciprofloxacin, control: ciprofloxacin

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and quinolines is capable of effectively preventing the occurrence of irritable bowel syndrome.

### Test Example 5-4: Lincomycin group antibiotics

### Drug: N-acetyl-D-glucosamine + lincomycin, control: lincomycin

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and lincomycin group antibiotic is capable of effectively preventing the occurrence of irritable bowel syndrome.

### Test Example 5-5: Chloramphenicol group antibiotics

### Drug: N-acetyl-D-glucosamine + chloramphenicol, control: chloramphenicol

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and chloramphenicol group antibiotic is capable of effectively preventing the occurrence of irritable bowel syndrome.

### Test Example 5-6: Tetracycline group antibiotics

### Drug: N-acetyl-D-glucosamine + tetracycline, control: tetracycline

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and tetracycline group antibiotic is capable of effectively preventing the occurrence of irritable bowel syndrome.

### Test Example 5-7: Cephalosporin group antibiotics

### Drug: N-acetyl-D-glucosamine + cefuroxime, control: cefuroxime

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and cephalosporin group antibiotic is capable of effectively preventing the occurrence of irritable bowel syndrome.

### Test Example 5-8: Penicillin group antibiotics

### Drug: N-acetyl-D-glucosamine + ampicillin, control: ampicillin

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and penicillin group antibiotic is capable of effectively preventing the occurrence of irritable bowel syndrome.

### Test Example 5-9: Other β-lactam antibiotics

### Drug: N-acetyl-D-glucosamine + cefoxitin, control: cefoxitin

Results: a large amount of CGCs appeared in the stools of the rats of control groups, and CGCs were still present in the rat stools after one week of drug withdrawal, while CGC was not detected in the stools of rats of test groups. As to the incidence rate of IBS in rats subjected to electric stimulation, intragastric administration with fructus zanthoxyli water, cold, constraint etc., it was 0 (0/30) for the rats of test groups, while it was 33% (10/30), 33% (10/30) and 33% (10/30) respectively for the rats of control groups.

Conclusion: the compound antibacterial preparation of N-acetyl-D-glucosamine and other β-lactam antibiotic is capable of effectively preventing the occurrence of irritable bowel syndrome.

## Claims

1. A compound antibacterial drug comprising N-acetyl-D-glucosamine and an antibiotic.

2. The compound antibacterial drug of claim 1, wherein said N-acetyl-D-glucosamine is the free base or a pharmaceutical acceptable salt thereof, and the antibiotic is selected from the group consisting of aminoglycoside antibiotics, macrolide group antibiotics, tetracyclines, quinolones, lincomycin group antibiotics, chloramphenicol group antibiotics, cephalosporin group antibiotics, penicillin group antibiotics, and β-lactam antibiotics other than penicillins and cephalosporins.

3. The compound antibacterial drug of claim 1, which is in the form of injections, tablets, capsules, and other mixed preparations.

4. Use of N-acetyl-D-glucosamine in the preparation of a compound antibacterial drug.

5. The use of claim 4, wherein said N-acetyl- D-glucosamine is the free base or a pharmaceutical acceptable salt thereof, and the antibiotic is selected from the group consisting of aminoglycoside antibiotics, macrolide group antibiotics, tetracyclines, quinolones, lincomycin group antibiotics, chloramphenicol group antibiotics, cephalosporin group antibiotics, penicillin group antibiotics, and *β*-lactam antibiotics other than penicillins and cephalosporins.

6. Use of N-acetyl-D-glucosamine in the preparation of the drugs for the treatment of irritable bowel syndrome, in vivo dysbacteriosis, functional disorder of intestine, etc.

7. The use of claim 6, wherein said N-acetyl- D-glucosamine is the free base or a pharmaceutical acceptable salt thereof, and the antibiotic is selected from the group consisting of aminoglycoside antibiotics, macrolide group antibiotics, tetracyclines, quinolones, lincomycin group antibiotics, chloramphenicol group antibiotics, cephalosporin group antibiotics, penicillin group antibiotics, and β-lactam antibiotics other than penicillins and cephalosporins.

8. A method of enhancing the treatment effect of antibiotics, comprising the step of a therapeutic effective amount of N-acetyl-D-glucosamine and a therapeutic effective amount of antibiotic to a patient in need.

9. The method of claim 8, wherein said N-acetyl- D-glucosamine is the free base or a pharmaceutical acceptable salt thereof, and the antibiotic is selected from the group consisting of aminoglycoside antibiotics, macrolide group antibiotics, tetracyclines, quinolones, lincomycin group antibiotics, chloramphenicol group antibiotics, cephalosporin group antibiotics, penicillin group antibiotics, and β-lactam antibiotics other than penicillins and cephalosporins.

10. A method for the treatment of bacterial infections or pathogenic proliferation that can be treated by antibiotics, comprising administering a therapeutically effective amount of N-acetyl-D-glucosamine and a therapeutically effective amount of antibiotic to a patient in need.
